# EUROPÄISCHE PATENTANMELDUNG

(11) **EP 3 207 924 A1**
(43) Veröffentlichungstag der Anmeldung: **23.08.2017**
(21) Anmeldenummer: 16156103.0
(22) Anmeldetag: 17.02.2016
(51) Int. Cl.: A61K 31/122, A61P 1/16, A61P 43/00

(54) **PHARMAZEUTISCHE ZUBEREITUNG ZUR HEMMUNG HUMANER CYP-ENZYME**

(71) Anmelder: Flaxan GmbH & Co. KG, 90482 Nürnberg (DE)
(72) Erfinder: Hellerbrand, Prof. Dr. Claus, 93053 Regensburg (DE); Mahli, Dr. Abdo, 93053 Regensburg (DE)
(74) Vertreter: Stippl, Hubert

(57) **Zusammenfassung**

Pharmazeutische Zubereitung umfassend eine Iso-alpha-Säure zur Hemmung humaner CYP-Enzyme, vorzugsweise des CYP2E1-Enzyms.

## Beschreibung

Die vorliegende Erfindung betrifft eine pharmazeutische Zubereitung zur Verminderten Bildung oder Hemmung der durch Alkohol gesteigerten Bildung humaner Cytochrome P450 (CYP)-Enzyme. Ferner betrifft die vorliegende Erfindung eine Zubereitung zur Behandlung und/oder Vorbeugung von Alkohol-induzierten Erkrankungen oder Schädigungen, insbesondere um Alkohol-induzierte Erkrankungen oder Schädigungen der Leber.

### Technologischer Hintergrund

Im Gegensatz zu anderen Nährstoffen wird Alkohol (Ethanol) im menschlichen Körper nicht gespeichert, sondern oxidativ abgebaut und schnell durch den Blutstrom im Gewebe verteilt. Der oxidative Abbau von Alkohol erfolgt hauptsächlich in der Leber, nur ein sehr kleiner Anteil der aufgenommenen Alkoholmenge wird unverändert über Nieren, Lungen oder die Haut ausgeschieden (Alkoholelimination).

Proteine der Cytochrom P450 (CYP) Familie sind körpereigene Enzyme und leisten einen wichtigen Beitrag bei der Verstoffwechselung (dem Abbau und der Ausscheidung) körpereigener wie auch von außen aufgenommener Stoffe. So beeinflussen sie z.B. auch wesentlich die Verstoffwechselung von Alkohol oder Arzneimitteln und beeinflussen so auch, wie lange jene im Köper verbleiben und wirksam sind.

In der Leberzelle stehen drei verschieden Enzymsysteme für den Alkoholabbau zur Verfügung. Das hierbei bedeutendste Enzym ist das humane Cytochrom CYP2E1 (bzw. P450 2E1). Dieses Enzym wird durch regelmäßigen Alkoholkonsum induziert. Der Alkoholabbau erfolgt in diesem Fall rascher. Das Enzym CYP2E1 hat hierbei die Eigenschaft, reaktive Sauerstoff-Intermediärprodukte mit toxischen Eigenschaften zu erzeugen. Diese wiederum führen zu entzündlichen Erkrankungen der Leber oder zu Schädigungen des Lebergewebes.

Bei Iso-alpha-Säuren handelt es sich um Hopfenbittersäuren, die aus dem Hopfen durch Extraktion, zum Beispiel CO₂-Extraktion, gewonnen werden und durch Hitzeeinwirkung isomerisiert werden. Iso-alpha-Säuren werden als wässrige Lösungen aus Erdalkalimetallsalzen, insbesondere Kaliumsalzen, der Iso-alpha-Säuren in Standardkonzentrationen ab ca. 25 % für die Anwendung in der Brauindustrie hergestellt. Sie dienen als Zusatz für den Brauprozess, um die Bittere im fertigen Bier genau einstellen zu können. Darüber hinaus wirkt die Zugabe von Iso-alpha-Säuren zu Bier antimikrobiell.

### Druckschriftlicher Stand der Technik

Aus der DE 10 2005 062 144 A1 ist es bekannt, Ingwer zur Inhibierung humaner CYP-Enzyme zu verwenden. Die Ingwerfraktion kann allein oder in Kombination mit Arzneimitteln verwendet werden, um die orale Bioverfügbarkeit und Pharmakokinetik von Wirkstoffen positiv zu beeinflussen.

Aus der DE 698 35 534 T3 ist es bekannt, zur Hemmung der Aktivität des Enzyms CPY2A6 ein Medikament mit einer wirksamen Menge von Tranylcypromin, Esculetin, Selegilin oder einem Naturprodukt ausgewählt aus Hypericum (Johanniskraut) oder Extrakten davon zu verwenden. Dies erfolgt vor dem Hintergrund, dass festgestellt wurde, dass das humane Cytochrom CYP die Metabolisierung von Nikotin inhibiert.

Aus "The Journal of Biological Chemistry", 2004, Band 279, Nr. 32, Seiten 33456-33462 ist es bekannt, dass Iso-alpha-Säuren PPARs Rezeptoren aktivieren sowie die Insulinresistenz reduzieren. Als Folge der Aktivierung von PPAR wird ein verstärkter Fettsäureabbau angesehen.

Aus der US 2006/0147568 A1 ist es bekannt, Iso-alpha-Säuren als Blutdruckreduzierende Substanz sowie als die Flexibilität von Blutgefäßen verbessernde Substanz einzusetzen.

### Aufgabe der vorliegenden Erfindung

Die Aufgabe der Erfindung besteht darin, eine neuartige Substanz zur Hemmung der Bildung humaner CYP-Enzyme zur Verfügung zu stellen.

### Lösung der Aufgabe

Die vorstehende Aufgabe wird durch die Merkmale des Anspruchs 1 gelöst. Zweckmäßige Ausgestaltungen der vorliegenden Erfindung werden in den Unteransprüchen beansprucht.

Es hat sich überraschend herausgestellt, dass Iso-alpha-Säure die Bildung humaner CYP-Enzyme hemmt. Dies führt in vorteilhafter Weise dazu, dass wenn humane CYP-Enzyme im Bedarfsfall gehemmt werden sollen, eine neue, auf pflanzlicher Basis beruhende, pharmakologische Zubereitung zur Verfügung steht. Iso-alpha-Säure fällt als Nebenprodukt bei der Hopfenverarbeitung an und ist daher vergleichsweise günstig in der Beschaffung.

Aufgrund der festgestellten Wirkung eignet sich die beanspruchte Zubereitung ganz besonders zur Anwendung bei der Therapie und/oder Prävention von Erkrankungen oder Schäden, vorzugsweise bei Lebererkrankungen oder Leberschäden, die aus schädlichen Stoffwechselprodukten der humanen CYP-Enzyme resultieren.

Da einige humane CYP-Enzyme (z. B. CYP2E1) vor allem bei der Metabolisierung von Alkohol (Ethanol) aktiviert werden, eignet sich die erfindungsgemäße Zubereitung zweckmäßigerweise vor allem zur Vermeidung von Lebererkrankungen oder Leberschäden aufgrund erhöhten Alkoholkonsums.

Da eine Alkohol-induzierte Erkrankung oder Schädigung der Leber in vielen Fällen zu Leberzirrhose oder Leberversagen führt, kann die erfindungsgemäße Zubereitung auch dazu dienen, Folgeerkrankungen, wie z. B. Leberzirrhose bzw. Leberversagen (z. B. nach exzessiven Alkoholkonsum), wirksam vorzubeugen.

Aufgrund der aufgefundenen Wirksamkeit von Iso-alpha-Säure kann die erfindungsgemäße Substanz auch vorteilhaft zur Erhöhung der Bioverfügbarkeit einer vorzugsweise oral zu applizierenden pharmazeutischen Verbindung eingesetzt werden, wobei es sich bei der pharmazeutischen Verbindung um eine solche handelt, die durch humane CYP-Enzyme metabolisiert wird, also im Körper abgebaut wird. Hierdurch kann erreicht werden, dass die Verabreichungsdosis pharmazeutischer Verbindungen erheblich reduziert und so auch schädliche Nebenwirkungen wirksam reduziert oder gar vermieden werden.

Die Iso-alpha-Säure (oder auch Isohumulone genannt) weist die nachstehende Grundstruktur auf:

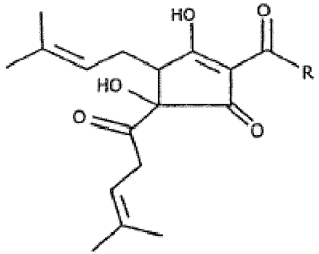

Vorzugsweise ist die Iso-alpha-Säure aus der Hopfenpflanze isoliert bzw. extrahiert. Beispielsweise handelt es sich um ein aus der Hopfenpflanze gewonnenes, vorzugsweise im CO2-Extraktionsverfahren extrahiertes, Alpha-Säure-Extrakt, welches durch Hitzebehandlung zu Iso-alpha-Säure isomerisiert wird. Der Begriff "Iso-alpha-Säure" umfasst unterschiedliche isomere Formen von Alpha- Säuren wie insbesondere z. B. Cis- und Trans-Isomere.

Vorteilhaft ist die Iso-alpha-Säure als Alkalimetallsalz, vorzugsweise Kaliumsalz, der Iso-alpha-Säure vorgesehen. Das Alkalimetallsalz der Iso-alpha-Säure ist stabil und für die Konfektionierung als stabile wässrige Lösung gut geeignet.

Die wässrige Lösung weist zweckmäßigerweise eine Konzentration an Alkalimetallsalz der Iso-alpha-Säure im Bereich von 30.0% bis 60% ± 1% (HPLC) auf.

Die Zubereitung kann geeigneter Weise auch als Nahrungsergänzungsmittel vorgesehen sein.

Für alle Anwendungen gilt, dass die Iso-alpha-Säure in einer therapeutisch wirksamen Dosierung in der Zubereitung vorliegt. Ferner umfasst die Zubereitung weitere Bestandteile, die dazu vorgesehen und geeignet sind, eine orale Aufnahme der Zubereitung zu ermöglichen.

Vorzugsweise handelt es sich bei den humanen CYP-Enzymen um CYP2E1, CYP7A1, CYP1A2, CYP27A1, CYP2A6, CYP51A1 oder CYP2B6.

Gemäß einer besonderen Ausgestaltung der vorliegenden Erfindung wird die Zubereitung bezogen auf den Reinanteil an Iso-alpha-Säure in einer Dosierung von 0,01 bis 500 mg/kg Körpergewicht, vorzugsweise 0,1 bis 100 mg/kg Körpergewicht, 0,01 bis 30 mg/kg Körpergewicht, vorzugsweise in einer Dosierung von 0,5 bis 50,0 mg/kg Körpergewicht, besonders vorzugsweise in einer Dosierung von 1,00 bis 10,0 mg/kg Körpergewicht verabreicht.

Zweckmäßigerweise umfasst die Zubereitung einen Zusatz zur Gewährleistung eines Geschmacks, vorzugsweise Fruchtgeschmacks, so dass der Eigenschmack der Iso-alpha-Säure abgeschwächt, kompensiert oder überlagert wird.

Die vorliegende Erfindung umfasst ferner eine Verwendung von Iso-alpha-Säure zur Hemmung humaner CYP-Enzyme, insbesondere von CYP2E1, CYP7A1, CYP1A2, CYP27A1, CYP2A6, CYP51A1 oder CYP2B6.

Zudem umfasst die vorliegende Erfindung eine Verwendung von Iso-alpha-Säure zur Erhöhung der Bioverfügbarkeit einer vorzugsweise oral zu applizierenden pharmazeutischen Verbindung, wobei es sich bei der pharmazeutischen Verbindung um eine solche handelt, die durch humane CYP-Enzyme, insbesondere durch CYP2E1, CYP7A1, CYP1A2, CYP27A1, CYP2A6, CYP51A1 oder CYP2B6, metabolisiert wird. Die Iso-alpha-Säure bzw. eine letztere umfassende Zubereitung wird vorzugsweise zusammen mit der pharmazeutischen Verbindung vorzugsweise oral appliziert bzw. zusammen mit der pharmazeutischen Verbindung in fester Form wie z. B. als Tablette, Kapsel, oder flüssiger Form konfektioniert.

### Beschreibung der Erfindung anhand von Untersuchungsergebnissen

Die Erfindung wird nachstehend anhand von Zeichnungsfiguren näher erläutert. Es zeigen:
- Fig. 1: verschiedene mRNA-Level von CYP 2E1 mit Hilfe einer quantitativen RT-TCR;
- Fig. 2: eine Analyse der CYP 2E1-Aktivität,
- Fig. 3: eine Darstellung der CYP 2E1-Proteinlevels in Lebergewebe,
- Fig. 4: verschiedene mRNA-Level von CYP 27A1,
- Fig. 5: verschiedene mRNA-Level von CYP 2B6,
- Fig. 6: verschiedene mRNA-Level von CYP 7A1,
- Fig. 7: verschiedene mRNA-Level von CYP 51A1,
- Fig.8: verschiedene mRNA-Level von CYP 1A2, sowie
- Fig. 9: verschiedene mRNA-Level von CYP 2A6.

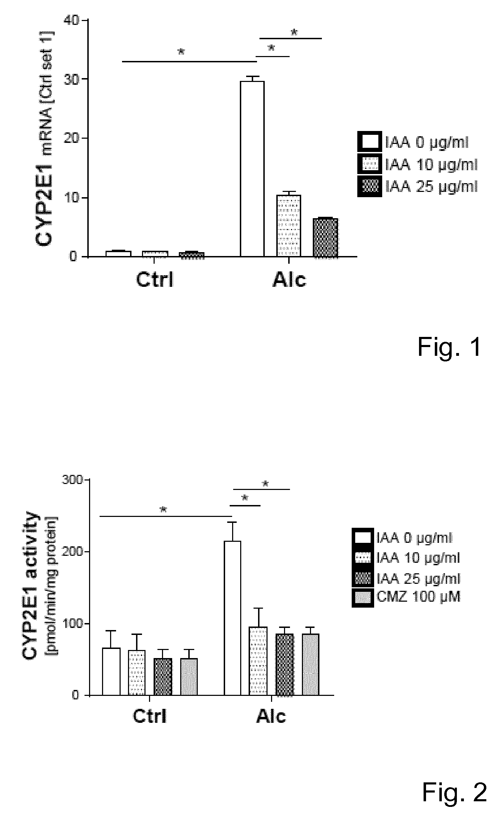

HepG2 E47-Zellen sind eine humane Hepatomazell-Linie, die das CYP 2E1-Enzym stabil exprimieren (bilden) und deshalb als valides Modell für primäre Hepatozyten (Leberzellen) verwendet werden können. Bei den Untersuchungen gemäß Fig. 1 wurden HepG2 E47-Zellen mit Iso-alpha-Säure über einen Zeitraum von 24 Stunden und unterschiedlicher Konzentration vorbehandelt. Bei den Konzentrationen handelt es sich um 0 µg/ml, 10 µg/ml sowie 25 µg/ml Iso-alpha-Säuren. Anschließend wurden die Zellen für einen zusätzlichen Zeitraum von 24 Stunden Alkohol (300 mM) ausgesetzt.

Die Ordinate in dem Diagramm von Fig. 1 zeigt die Transkriptionsmenge an mRNA und steht daher für die Menge an mRNA des CYP 2E1-Enzyms. mRNA ist die Vorlage für die Proteinbildung und zeigt somit die Menge an CYP 2E1 mRNA auch die Menge an CYP 2E1 Protein an.

Die Untersuchungen ergaben bei fehlender Zugabe von Iso-alpha-Säuren in Gegenwart von Alkohol einen sehr ausgeprägten Anstieg der CYP 2E1 mRNA im Vergleich zu Kontrollzellen (ohne Alkohol), der durch Alkohol induziert wird. Ein Vergleich der weißen Balken (Ctrl und Alc) zeigt, dass Alkohol CYP 2E1 etwa 30-fach induziert ist. Überraschenderweise hat sich gezeigt, dass bereits bei den mit einer Konzentration von 10 µg/ml behandelten Zellen die CYP 2E1-mRNA schon mehr als um die Hälfte reduziert ist bzw. der Alkohol induzierte Anstieg gehemmt ist. Die Iso-alpha-Säure verhindert also, dass durch Alkohol CYP 2E1-mRNA induziert wird.

Es zeigt sich auch, dass der Effekt dosierungsabhängig ist. Bei einer Zugabe einer höher dosierten Iso-alpha-Säure (IAA 25 µg/ml) wird die CYP 2E1-mRNA Menge sogar noch weiter herabgesetzt. Das statistische Signifikanzniveau war < 0,05 verglichen mit der entsprechenden Kontrollmessung.

Es wird somit anhand der Ergebnisse der Hepatomazell-Linie deutlich, dass durch Verabreichung von Iso-alpha-Säure die Bildung des Enzyms CYP 2E1 trotz hohen Alkoholgehalts sehr wirksam reduziert bzw. gehemmt werden kann.

Die Darstellung gemäß Fig. 2 zeigt eine Analyse der Aktivität des CYP 2E1-Enzyms. Zum Vergleich der Iso-alpha-Säure Wirkung wurde hier Chlormethiazol (CMZ), eingesetzt, ein spezifischen CYP 2E1-Aktivitätshemmer.

Die Darstellung gemäß Fig. 2 zeigt, dass bei Verabreichung von Iso-alpha-Säure in Konzentrationen von 10 µg/ml bzw. 25 µg/ml die Alkohol-induzierte Steigerung der Aktivität des CYP 2E1-Enzyms nahezu in der gleichen Weise reduziert werden kann, wie dies mit dem bekannten CYP 2E1-Hemmer (Chlormethiazole) erzielt werden kann. Das statistische Signifikanzniveau war < 0,05 verglichen mit der entsprechenden Kontrollmessung.

Neben den Untersuchungen im Rahmen der Hepatomazell-Linie wurden auch *in vivo* Untersuchungen an Mäusen durchgeführt. Iso-alpha-Säure wurde Mäusen (C57BI/6N Mäuse) in der Konzentration 100 mg/kg Körpergewicht durch orale Verabreichung zugeführt. Kontroll-Mäuse (Ctrl) erhielten lediglich Trägerstoff (Vehicle). Nach drei Stunden wurde allen Mäusen durch orale Verabreichung 6g/kg Körpergewicht Alkohol verabreicht. Nach 12 Stunden wurden die Mäuse getötet und deren Leber für nachfolgende Untersuchungen schockgefroren.

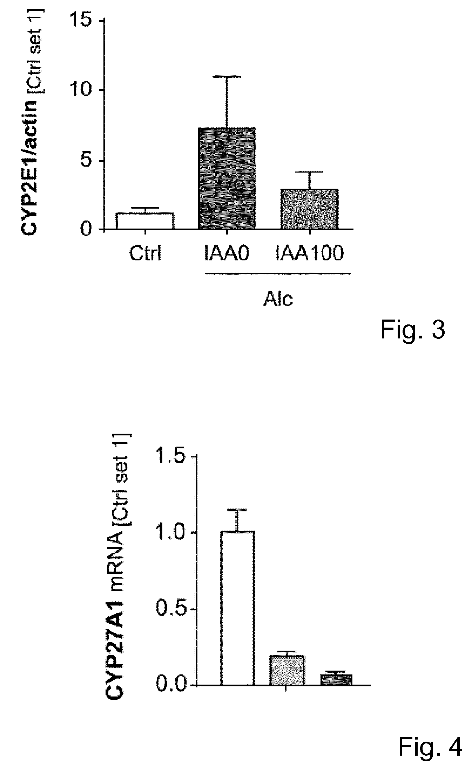

Fig. 3 zeigt die CYP 2E1-Protein-Level in Lebergewebe. Beta-Aktin diente als Kontrolle dafür, dass in der Analyse jeweils gleiche Mengen an Gesamtprotein eingesetzt wurden. Man erkennt, dass der mittlere Balken (IAA0), betreffend die Lebergewebeproben von Mäusen, die lediglich Alkohol ausgesetzt worden sind, einen sehr viel höheren Proteinlevel aufweist, als der rechte Balken (IAA100), betreffend die Lebergewebeproben von Mäusen, denen Alkohol in Verbindung mit Iso-alpha-Säuren verabreicht worden ist. Auch hier ist auffällig, dass die Bildung des Enzyms CYP 2E1 trotz hohen Alkoholgehalts um mehr als die Hälfte, also sehr wirksam, reduziert bzw. gehemmt werden kann.

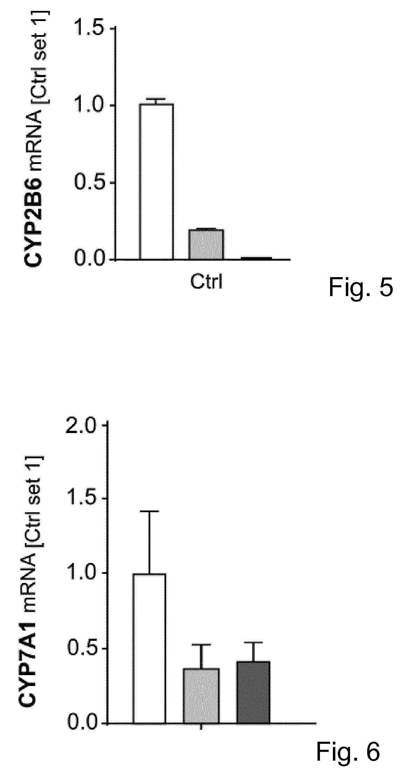

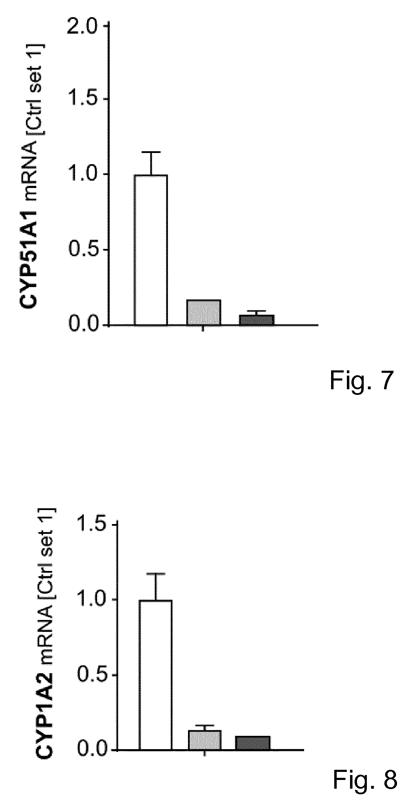

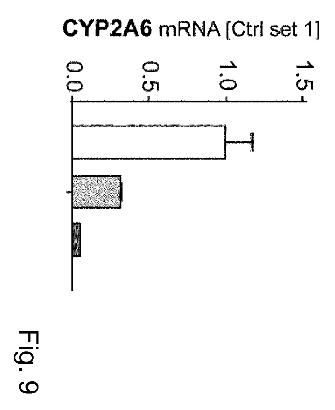

Die Wirksamkeit von Iso-alpha-Säuren ist jedoch nicht nur auf das CYP 2E1-Enzym beschränkt, sondern liegt bei einer Vielzahl von humanen CYP-Enzymen vor, wie dies aus den Untersuchungsergebnissen gemäß den Fig. 4-9 ersichtlich ist. Hierbei handelt es sich um die Enzyme CYP 27A1 gemäß Fig. 4, CYP 2B6 gemäß Fig. 5, CYP 7A1 gemäß Fig. 6, CYP 51A1 gemäß Fig. 7, CYP 1A2 gemäß Fig. 8 sowie CYP 2A6 gemäß Fig. 9. Bei allen zeigt sich eine erhebliche Reduzierung beziehungsweise Hemmung der Bildung des betreffenden Enzyms durch Verabreichung von Iso-alpha-Säure. Bei allen Enzymen bis auf das Enzym CYP 7A1 gemäß Fig. 6 zeigt sich auch, dass der Effekt dosierungsabhängig ist, also mit höher dosierter Iso-alpha-Säure die Bildung des betreffenden Enzyms sogar noch zusätzlich herabgesetzt werden kann.

In den Fig. 4-9 wurde die Wirkung von Iso-alpha-Säure auf primäre humane Hepatozyten (PHH) untersucht. Untersucht wurde hierbei 2 verschiedene Iso-alpha-Säure Konzentrationen (10 µg/ml: hellgraue Balkenfarbe) und 25 µg/ml: dunkelgraue Balkenfarbe) im Vergleich zu Kontrollzellen (weiße Balkenfarbe). Angezeigt sind jeweils auf der Ordinate die Mengen an mRNA-Mengen der jeweiligen CYP-Enzyme.

Die Isolierung und Zellkultivierung der primären humanen Hepatozyten (PHH) wurde wie in Weiss TS et al., Cell Prolif 2002; 35 (5):257-67 durchgeführt. Die PHH wurden mit seriellen Konzentrationen von Iso-alpha-Säure (bis zu 25 µg/ml) oder äquivalenten Konzentrationen des Lösungsmittels (Alkohol; Ctrl) für 12 Stunden inkubiert. Die Analyse der mRNA-Level der Cytochrome P450 (CYPs)-Ausbildung wurde unter Zuhilfenahme der quantitativen RT-PCR durchgeführt.

Bei allen Untersuchungen wurde eine im Handel erhältliche Iso-alpha-Säure der Marke ISOHOP® (Stand Iso Juli 2010) der Barth-Haas Group verwendet. Es handelt sich hierbei um eine standardisierte wässrige Lösung aus Kaliumsalzen der Iso-alpha-Säuren mit einer Standardkonzentration von 30,0% ± 0,5% Iso-alpha-Säuren (HPLC) sowie einem pH-Wert von 8,0 - 10,0. Diese Iso-alpha-Säure ist löslich in Wasser sowie in Alkohol. Die Iso-alpha-Säure wurde im Rahmen einer CO₂-Extraktion gewonnen. 100mg/ml Iso-alpha-Säure-Vorrat wurde aus ISOHOP® durch Lösen in Ethanol (99%) hergestellt.

Ausdrücklich vom Offenbarungsinhalt mit umfasst sind auch Unterkombinationen von einzelnen Merkmalen der beschriebenen pharmazeutischen Zubereitung und/oder der beschriebenen Verfahren.

## Patentansprüche

1. Pharmazeutische Zubereitung umfassend eine Iso-alpha-Säure zur Hemmung humaner CYP-Enzyme.

2. Zubereitung nach Anspruch 1 zur Anwendung bei der Behandlung und/oder Vorbeugung von Erkrankungen oder Schäden, vorzugsweise um Lebererkrankungen oder Leberschäden, die jeweils aus Stoffwechselprodukten der CYP-Enzyme resultieren.

3. Zubereitung nach Anspruch 2, **dadurch gekennzeichnet, dass** es sich bei den Lebererkrankungen oder Leberschäden um Alkohol-induzierte Erkrankungen oder Schädigungen der Leber handelt.

4. Zubereitung nach Anspruch 2 oder 3, **dadurch gekennzeichnet, dass** die Zubereitung der Vorbeugung des Fortschreitens der Lebererkrankung oder Leberschädigung dient.

5. Zubereitung nach Anspruch 1 zur Erhöhung der Bioverfügbarkeit einer vorzugsweise oral zu applizierenden pharmazeutischen Verbindung, wobei es sich bei der pharmazeutischen Verbindung um eine solche handelt, die durch humane CYP-Enzyme metabolisierbar ist.

6. Zubereitung nach mindestens einem der vorhergehenden Ansprüche, **dadurch gekennzeichnet, dass** als Iso-alpha-Säure eine aus Hopfen extrahierte und isomerisierte Alpha-Säure vorgesehen ist.

7. Zubereitung nach mindestens einem der vorhergehenden Ansprüche, **dadurch gekennzeichnet, dass** die Iso-alpha-Säure(n) die nachstehende Grundstruktur aufweist:

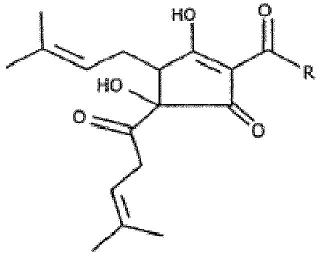

8. Zubereitung nach mindestens einem der vorhergehenden Ansprüche, **dadurch gekennzeichnet, dass** eine wässrige Lösung eines Alkalimetallsalzes der Iso-alpha-Säure vorgesehen ist.

9. Zubereitung nach Anspruch **9, dadurch gekennzeichnet, dass** die wässrige Lösung eine Konzentration an Alkalimetallsalz der Iso-alpha-Säure im Bereich von 30.0% bis 60% ± 1% (HPLC) aufweist.

10. Zubereitung nach mindestens einem der vorhergehenden Ansprüche, **dadurch gekennzeichnet, dass** es sich bei den humanen CYP-Enzymen um CYP 2E1, CYP 7A1, CYP 1A2, CYP 27A1, CYP 2A6, CY P51A1 oder CYP 2B6 handelt.

11. Zubereitung nach mindestens einem der vorhergehenden Ansprüche, **dadurch gekennzeichnet, dass** die Zubereitung in einer Dosierung von 0,01 bis 500 mg/kg Körpergewicht, vorzugsweise in einer Dosierung von 0,1 bis 100,0 mg/kg Körpergewicht, vorzugsweise in einer Dosierung von 0,5 bis 50,0 mg/kg Körpergewicht, besonders vorzugsweise in einer Dosierung von 1 bis 10,0 mg/kg Körpergewicht vorgesehen ist.

12. Zubereitung nach mindestens einem der vorhergehenden Ansprüche, **dadurch gekennzeichnet, dass** die Zubereitung als Nahrungsergänzungsmittel vorgesehen ist.

13. Verwendung von Iso-alpha-Säure zur Hemmung humaner CYP-Enzyme, insbesondere von CYP2E1, CYP7A1, CYP1A2, CYP27A1, CYP2A6, CYP51A1 oder CYP2B6.

14. Verwendung von Iso-alpha-Säure zur Erhöhung der Bioverfügbarkeit einer vorzugsweise oral zu applizierenden pharmazeutischen Verbindung, wobei es sich bei der pharmazeutischen Verbindung um eine solche handelt, die durch humane CYP-Enzyme, insbesondere durch CYP2E1, CYP7A1, CYP1A2, CYP27A1, CYP2A6, CYP51A1 oder CYP2B6, metabolisiert wird.
